# EUROPEAN PATENT APPLICATION

(11) **EP 4 582 007 A1**
(43) Date of publication of application: **09.07.2025**
(21) Application number: 24150029.7
(22) Date of filing: 02.01.2024
(51) Int. Cl.: A61B 1/00, A61B 1/05, A61B 1/24, A61B 5/00

(54) **ORAL SCANNER SYSTEM**

(71) Applicant: Braun GmbH, 61476 Kronberg im Taunus (DE)
(72) Inventor: ENGELMOHR, Reiner, 61476 Kronberg (DE); BOSS, Rüdiger, 61476 Kronberg (DE); ALBAY, Devran, 61476 Kronberg (DE); LANGHAMMER, Dominik Heinz, 61476 Kronberg (DE); VETTER, Ingo, 61476 Kronberg (DE); CHENG, Kai-Ju, Taoyuan City, 33377 (CN); HSIEH, Hsin-Lun, Taoyuan City, 33377 (CN); TING, Chin-Yuan, Taoyuan City, 33377 (CN); LU, Tsung-Hsin, Taoyuan City, 33377 (CN); CHANG, Chin-Kang, Taoyuan City, 33377 (CN); HUANG, Chao-Ching, Taoyuan City, 33377 (CN); LIN, Wan-Chi, Taoyuan City, 33377 (CN); CHEN, Tao-Feng, Taoyuan City, 33377 (CN)
(74) Representative: P&G Patent Germany

(57) **Abstract**

The present disclosure is concerned with an oral scanner system having an oral scanner having a handle, a head, a camera having a light inlet provided at the head, the camera having a light-sensitive sensor element array arranged for outputting at least one signal indicative of light intensity on at least one of a plurality of light-sensitive sensor elements of the light-sensitive sensor element array, a spacer attachment being detachably attached to the head, the spacer attachment enabling a constant distance between a scanned object and the light inlet of the camera, and a user-operable input element for initiating a scanning procedure by the oral scanner, and a processor being arranged to receive the at least one signal from the camera in response to an operation of the user-operable input element and/or automatically at least once during operation of the oral scanner and further being arranged to determine, based on an analysis of the at least one signal, an attachment state of the spacer attachment.

## Description

### FIELD OF THE INVENTION

The present disclosure is concerned with an oral scanner system that comprises an oral scanner and a processor, where the processor may be provided in the oral scanner or in a separate device. The oral scanner comprises a camera and a spacer attachment.

### BACKGROUND OF THE INVENTION

A dental diagnostic camera and a spacer attachment for detachable connection to a head region of the diagnostic camera is known. In such a known diagnostic camera, the head region contains a camera unit with optical and electronic components such as lens optics and an image-recording device and further a light source. The spacer attachment has a spacer portion having a free end portion, which spacer portion is arranged on a coupling portion which can be detachably arranged on the housing. The free end portion indicates to the user how closely the diagnostic camera has to be advanced towards an object such as a tooth in order to provide a sharp image. Document US 2010/0238279 A1 generally describes such a diagnostic camera.

It is a general object to improve an oral scanner system, preferably to improve an oral scanner system with respect to a more reliable usage.

### SUMMARY OF THE INVENTION

In accordance with one aspect there is provided an oral scanner system comprising an oral scanner having a handle, a head, a camera having a light inlet provided at the head, the camera comprising a light-sensitive sensor element array arranged for outputting at least one signal indicative of light intensity on at least one of a plurality of light-sensitive sensor elements of the light-sensitive sensor element array, a spacer attachment being detachably attached to the head, the spacer attachment enabling a constant distance between a scanned object and the light inlet of the camera, and a user-operable input element for initiating a scanning procedure by the oral scanner, and a processor being arranged to receive the at least one signal from the camera in response to an operation of the user-operable input element and/or automatically at least once during operation of the oral scanner and further being arranged to determine, based on an analysis of the at least one signal, an attachment state of the spacer attachment.

In accordance with one aspect there is provided an oral scanner system comprising an oral scanner having a handle, a head, an oral scanner attachment being detachably attached to the head, wherein the head comprises a first interacting element and the oral scanner attachment comprises a second interacting element, the first interacting element and the second interacting element being arranged so that the first interacting element provides at least one signal being indicative of an attachment state of the oral scanner attachment in dependence on the presence of the second interacting element, and processor being arranged to receive the at least one signal from the first interacting element and to determine the attachment state of the oral scanner attachment and preferably further being arranged to initiate an action in dependence on the determined attachment state such as an indication of the determined attachment state via an indicator such as a visual indicator, an acoustic indicator and/or a tactile indicator.

### BRIEF DESCRIPTION OF THE DRAWINGS

The present disclosure will be further elucidated by a detailed description of example embodiments and with reference to figures. In the figures
- Fig. 1: is a depiction of an example oral scanner system comprising an oral scanner and a processor disposed in the oral scanner;
- Fig. 2: is a depiction of an example oral scanner system comprising an oral scanner and a separate device in which the processor is disposed;
- Fig. 3A: is a depiction of an example connector portion of a spacer attachment for attachment at the head of an oral scanner;
- Fig. 3B: is a depiction of an example contact portion of a spacer attachment, where it should be understood that the connector portion of Fig. 3A and the contact portion may be non-detachably connected;
- Fig. 4A: is a head portion of an example oral scanner without a spacer attachment;
- Fig. 4B: is a head portion of an example oral scanner with an example spacer attachment being attached to the head portion;
- Fig. 5A: is a schematic cross-sectional depiction of a camera of a head of an example scanner having a first field of view and a second field of view where at least a portion of the spacer attachment is visible in the second field of view;
- Fig. 5B: is a schematic top view indicating the field of view and the maximum field of view, where it is indicated that portions of the spacer attachment are visible in the corners of the maximum field of view;
- Fig. 6: is a depiction of a spacer attachment comprising a visually detectable identifier, which here is a QR code; and
- Fig. 7: is a cross-sectional cut through a head portion of an example oral scanner with a spacer attachment attached to the head.

### DETAILED DESCRIPTION OF THE INVENTION

An oral scanner system may be used by a human subject such as a user or a dentist or a dental assistant to image at least a portion of an oral cavity, specifically of a dentition. For some purposes it may be sufficient to just image said portion of the oral cavity by, e.g., using a white light source. For other purposes it may be sensible to use a light source with a narrow wavelength range to generate diagnostically relevant images, e.g., images of fluorescence light. In the present disclosure, both such purposes are generally considered and while the focus is here on a home-use device, it shall not be excluded that the oral scanner system is intended for professional use.

Light having a wavelength of about 405 nm causes the emission of fluorescence light from oral cavity tissue. Fluorescence light emitted from oral cavity tissue, mostly tooth enamel and dental plaque, excited by 405 nm blue light shows a broad wavelength distribution above 405 nm, where tooth enamel mainly emits greenish autofluorescence light and dental plaque typically emits red fluorescence light in a main emission range of between 600 nm and 650 nm, which fluorescence light is believed to have a microbiological origin. A camera having several color channels such as an RGB camera, allows automated detection of dental plaque by identifying the red fluorescence emission. The here described mechanism is known as QLF (quantitative light-induced fluorescence) and is used by professional devices. The present disclosure focuses on an oral scanning system comprising an oral scanner for performing any type of scanning procedure including white light imaging and QLF analysis.

An oral scanner system in accordance with the present disclosure comprises an oral scanner and a processor. By this terminology it shall not be excluded that the processor may be provided in or at the oral scanner, e.g., the processor may be provided within a housing of the oral scanner and may essentially be realized as an integrated circuit, e.g., as an IC chip on a PCB of the oral scanner. But the processor may alternatively be remotely provided, e.g., a separate device may comprise or realize the processor or a cloud computer may comprise or realize the processor. The above shall not exclude that the processor is realized by a first processor and a second processor, where the first processor and the second processor are provided at different locations, e.g., the first processor may be provided in or at the oral scanner and the second processor may be provided in a separate device or the first processor may be provided in a separate device and the second processor may be provided by a cloud computer etc. In such a scenario, the first processor may perform first processing steps and the second processor may perform second processing steps.

An oral scanner in accordance with the present disclosure comprises a handle and a head. The handle may be arranged for being held in the hand of a user and the head may be arranged for insertion into an oral cavity of a human subject and for being moved around typical objects that are present in an oral cavity such as teeth of the dentition and a tongue to scan at least a portion of the oral cavity. The oral scanner generally comprises a first interacting element that specifically may be a camera being arranged for performing a scanning procedure, which typically is an oral scanning procedure, where the term scanning procedure here refers to a procedure in which one image or a sequence of images is captured by the camera. The camera comprises a light inlet that is provided at the head of the oral scanner. This may allow to dispose, e.g., a light-sensitive sensor element array of the camera in the handle and to guide the light from the light inlet to the light-sensitive sensor element array by means of optical elements such as one or more lenses, one or more mirrors and/or one or more prisms etc. Further, a user-operable input element may be provided at the oral scanner that may be connected with the processor so that upon operation by the user a scanning procedure may be initiated by the processor.

The oral scanner also generally comprises an oral scanner attachment that specifically may be a detachable spacer attachment. The spacer attachment is arranged to enable a scanning procedure with an essentially constant distance between the object or the objects that are being scanned and the light inlet of the camera. A distance piece or contact portion - both terms may be used herein for the same feature - of the spacer attachment may stay in contact with the object being scanned - specifically the outer surface of the object - to maintain a constant distance. The camera may have a focal length that creates sharp images of objects that have a distance to the light inlet of the camera as is defined by the distance piece. The distance piece may be realized as a closed wall element that surrounds the light inlet of the camera so that the closed wall element effectively blocks ambient light from illuminating the currently scanned object. The distance piece or contact portion may be realized as a closed wall defining an aperture, further preferably wherein the closed wall is ring-shaped, O-shaped, lozenge-shaped, ellipsoidal, oval or essentially rectangular. The distance piece may be made from a relatively soft material such as a thermoplastic elastomer that may have a Shore A hardness of below 80, preferably below 70 and further preferably of below 60.

The spacer attachment may be detachable to allow replacing the spacer attachment when it is worn out or to allow changing the spacer attachments if different spacer attachments are used by different users of the oral scanner. The spacer attachment may also be detachable to improve accessibility to parts of the oral scanner that benefit from regular cleaning such as a window covering the light inlet of the camera. Also, the detachable spacer attachment may benefit from regular cleaning, which cleaning becomes simpler when the spacer attachment is detachable. E.g., the spacer attachment may be immersed into a cleaning liquid to clean and to potentially sterilize it.

The spacer attachment may further comprise a connector portion, preferably a U-shaped connector portion, which connector portion is arranged to become into engagement with a respective corresponding connector portion of the head. A connection direction for connecting the connector portion may essentially coincide with a longitudinal extension direction of the oral scanner. The connector portion of the spacer attachment may comprise at least a first mechanical connector element for a form-fit connection with a corresponding first mechanical connector element provided at the connector portion of the head. The first mechanical connector element of the spacer attachment may be a groove or a circumferential projection and the respective first mechanical connector element of the connector portion of the head may then be complementary to the groove or projection. The first mechanical connector element may extend along the U-shaped connector portion of the distance element and thus may extend in a U-shaped manner. The connector portion of the distance element may comprise at least a second mechanical connector element that is arranged for getting into a mechanical engagement, e.g., a snapping-type mechanical engagement, with a respective second mechanical connector element provided at the connector portion of the head when the spacer attachment reaches its intended attachment position in an attachment procedure. The mechanical partners may be arranged to allow a repeated attachment and detachment of the spacer attachment to and from the head. The connector portion may be made from a relatively hard but still elastic material such as a thermoplastic material, e.g., a polycarbonate or polypropylene or an acrylic etc. having a Shore D hardness of at least 50, preferably at least 60 and further preferably at least 70.

The spacer attachment may be realized as a two-component plastic part that may be injection molded by two consecutive plastic injection molding steps as is basically known in the art. The material of the connector portion and the material of the contact portion may be chosen such that they establish a chemical and/or physical bond in the injection molding process. The bonding may be enhanced by an undercut structure at the border between the two materials.

It is generally considered that the spacer attachment as described herein may represent an independent aspect of the present disclosure.

The head of the scanner may also comprise at least one light outlet allowing light from at least a first light source disposed in the scanner, specifically in the head of the scanner, to illuminate the object to be scanned. The light outlet may be located within the closed wall element. The distance piece can thus solve two objects, namely, to maintain a constant distance during the scanning procedure and to block ambient light from reaching the object surfaces to be scanned. The latter being of particular benefit for embodiments where the light emitted by the at least first light source shall mainly be responsible for the light impinging onto the light-sensitive sensor element array.

The oral scanner may comprise a second light source and potentially further light sources. The different light sources may use the same light outlet, or each light source may have its own associated light outlet. The first light source may emit light having a first wavelength or having a first wavelength range and the second light source may emit light having a second wavelength different to the first wavelength or light having a second wavelength range that does not or only partly overlap with the first wavelength or first wavelength range, respectively, of the first light source. Additionally or alternatively, the first and the second light source may be arranged to emit different light intensities. But this shall not exclude that a first and a second light source are provided to emit light of essentially the same wavelength or having the same wavelength range and also of essentially the same intensity. As one example, the first light source may emit light having a wavelength of or comprising a dominant wavelength of about 405 nm and the second light source may emit "white" light, i.e., light essentially covering the complete visual wavelength range between 400 nm and 700 nm or comprising several dominant wavelengths so that a human may consider the emitted light as essentially white. The light sources are not limited to light sources that emit light in the visual range and any light source may emit in the IR or UV wavelength range or at least comprise wavelengths in these areas as well. The first and/or second light source (and any further light source) may be realized by a light emitting diode (LED), but also other light sources are contemplated, e.g., laser diodes, conventional light bulbs - specifically incandescent light bulbs, halogen light sources, gas discharge lamps, arc lamps etc.

The camera comprises an array of light-sensitive sensor elements, where each light-sensitive sensor element may be arranged to output a signal indicative of a light intensity impinging onto a light-sensitive area of the light-sensitive sensor element. While each of the light-sensitive sensor elements may have an individual sensitivity range, i.e., individual wavelength sensitivity, the light-sensitive sensor element array may typically comprise light-sensitive sensor elements that all have about the same light sensitivity (ignoring differences in gain and the like as are typical). The array of light-sensitive sensor elements may be realized as a regular M time N array even though this shall not exclude that the light-sensitive sensor elements may be arranged in a different manner. The array of light-sensitive sensor elements may be realized as a CCD chip or a CMOS chip as are typically used in digital cameras. The number of light-sensitive sensor elements may be chosen in accordance with the needs of the scanning procedure and the processing power of the processor. A resolution of 640 time 480 may be one choice but essentially all other resolutions are conceivable, e.g., the camera may be a 4K camera having a 3840 times 2160 resolution.

In the context of the present application, a light-sensitive sensor element encompasses RGB sensor elements, i.e., each RGB-type light-sensitive sensor element would then deliver three signals that relate to the R (red), G (green) and B (blue) color channels.

The camera of the oral scanner may comprise further optical elements such as at least one lens to focus the light onto the array of light-sensitive sensor elements, even though this shall not exclude that the camera is realized as a pinhole camera. In some embodiments, a lens in front of the light-sensitive sensor array may be exchangeable so that two different fields of view can be achieved. The camera may also comprise at least one mirror. Further, the camera may comprise at least one filter to selectively absorb light of a certain wavelength or light of a certain wavelength range or light below or above a certain wavelength. The filter may be fixed or may be moveable, i.e., the filter may be arranged for being moved into and out of the light path of the camera. Several filters may be provided to allow selective filtering of the light that should reach the array of light-sensitive sensor elements. The filter may be a long-pass filter, a short-pass filter, a band-pass filter, or a monochromatic filter. The filter may be realized as a colored filter or as a dichroic filter.

The first light source may be a narrow-band light source such as an LED. The narrow-band light source may emit light in the range of between 390 nm and 410 nm (FWHM) such that a wavelength of about 405 nm is close to the dominant wavelength of the LED. Light of around 405 nm causes fluorescence light to be emitted by tooth enamel and by plaque. A filter may then be used that transmits only light having a wavelength above about 430 nm, preferably a cut-off filter having a cut-off wavelength of 450 nm allowing light of greater wavelength to pass towards the light-sensitive sensor element array to discard reflected light from the first light source and to only measure fluorescence light.

The camera may be realized by a camera module as is available, e.g., from Bison Electronics Inc., Taiwan. Without any limitation, the camera module may comprise a light-sensitive sensor array having an M times N pixel count of 1976 times 1200 (i.e., a 2.4-megapixel chip) realized in CMOS technology, but not all pixels may necessarily be used for capturing images during a scan. The camera module may comprise a lens having a 12.5 mm focal length so that sharp images can be captured of objects close to the camera.

In some embodiments, the processor is arranged to receive at least one signal from the camera, which signal is indicative of a light intensity of the light impinging onto at least one light-sensitive sensor element of the array of light-sensitive sensor elements of the camera. The processor is then arranged to determine an attachment state of the oral scanner attachment / the spacer attachment based on an analysis of the at least one signal. The camera may send the at least one signal in response to a user-operable input element being operated to initiate a scanning procedure and/or the at least one signal may be automatically outputted by the camera and received by the processor at least once during a scanning procedure. This automatic outputting may happen in accordance with a predetermined timeline or schedule, e.g., the at least one signal may be outputted a certain period after the scanning procedure was initiated or the at least one signal may be outputted in a regular manner, e.g., every two seconds or the like. The processor may be arranged to determine the attachment state by comparing a value of the at least one signal with at least one threshold value or by determining whether the value of the at least one signal of the at least one light-sensitive sensor element lies within a predetermined signal value range or by determining whether the value of the at least one signal differs from a value of at least one further signal received from the camera. The processor may be arranged to initiate an action of the oral scanner system in dependance on the determined attachment state of the oral scanner attachment / the spacer attachment such as to inhibit the scanning procedure if the analysis of the at least one signal reveals that the spacer attachment is not attached.

If the spacer attachment is not attached, the camera may not be kept at a constant distance to the object to be scanned. The oral scanner system may thus be arranged to communicate to the user an attachment state of the spacer attachment is missing by any user noticeable signal, e.g., by a visual signal, an acoustic signal, or a tactile signal. The processor may thus be arranged to initiate as an action in response to the determination of the attachment state an indication of the determined attachment state of the oral scanner attachment / the spacer attachment via an indicator such as a visual indicator, an acoustic indicator and/or a tactile indicator. A visual signal may be provided by at least one light source or a display, an acoustic signal may be provided by a loudspeaker or a buzzer or the like, and a tactile signal may be provided by vibration or electric current flow.

From a more general point of view, it is considered that an attachment state of the oral scanner attachment is determined based on first and second interacting elements provided as the head and the spacer attachment. The first interacting element is at least partially provided at the head and is arranged to output at least one signal indicative of the attachment state of the oral scanner attachment, i.e., the signal allows at least to derive that the oral scanner attachment is not attached to the head or in other words to derive the lack of presence of the oral scanner attachment. Various first and second interacting elements are contemplated, and the below list is understood to be not limiting and not complete and shall be a starting point for a skilled person to understand the concept:
a. A mechanical switch at the head realizing the first interacting element that is actuated by a mechanical structure at the oral scanner attachment realizing the second interacting element.
b. An RFID reader at the head realizing the first interacting element and a RFID tag at the oral scanner attachment realizing the second interacting element.
c. A light emitter such as an LED and a light detector such as a photodiode at the head together realizing the first interacting element and a reflecting, potentially selectively reflecting structure at the oral scanner attachment realizing the second interacting element.
d. A reed switch at the head realizing the first interacting element and a magnet for closing the reed switch at the oral scanner attachment realizing the second interacting element.
e. A capacitive switch or resistive touch switch or piezo touch switch may be provided at the head realizing the first interacting element and a respective portion of the spacer attachment realizing the second interacting element may cause the switch to be actuated.

With reference to the previous discussion, it is to be understood that the camera provided at the oral scanner having a light inlet at the head is realizing the first interacting element and a portion of the spacer attachment extending into a field of view of the camera is realizing the second interacting element.

While the first and second interacting elements may automatically, i.e., without a specific trigger, or continuously provide the at least one presence state signal to the processor, it shall be understood that the at least one attachment state signal may only be transmitted from the first interacting element to the processor when a user-operable input element of the oral scanner is operated to initiate a scanning procedure.

In any case, when the processor of the oral scanner system is provided at or within the oral scanner, the processor may be directly connected with the first interacting element, e.g., the camera, by electrical leads. When the processor is provided by a separate device, the oral scanner may comprise a transmitter or transceiver for wirelessly transmitting the signal from the first interacting element such as the camera to the separate device. The separate device may then comprise a receiver or transceiver for wirelessly receiving the signal.

It has been described that the attachment state of the oral scanner attachment can be determined from a signal of a first interacting element provided at the head that interacts with a second interacting element provided at the spacer attachment. It shall now be focused on a camera realizing the first interacting element and the oral scanner attachment, specifically a contact portion of a spacer attachment realizing the oral scanner attachment as the second interacting element.

In simple words, the camera may have a field of view into which at least a portion of the spacer attachment, preferably a portion of the contact portion extends. That means that at least one outer light-sensitive sensor element of the array of light-sensitive sensor elements images the mentioned portion of the spacer attachment extending into the field of view of the camera. Typically, the portion of the spacer attachment extending into the maximum field of view of the camera may be designed such that more than one light-sensitive sensor element images this portion, e.g., 2 or 3 or 4 or 5 or 10 or 20 or 30 or 50 or 100 or more light-sensitive sensor elements may image this portion. Preferably two or even up to four corners or areas of the array of light-sensitive sensor element may image respective portions of the spacer attachment extending into the field of view of the camera.

While it shall not be excluded that the oral scanner system also uses the mentioned field of view of the camera for the scanning procedure, it is contemplated that the camera has a first field of view and a second field of view. The latter may also be called a regular field of view that is used for the scanning procedure, where the regular field of view is provided such that the portion of the spacer attachment extending into the first field of view of the camera does not extend into the second or regular field of view. The first and second field of views may be achieved by exchangeable lenses so that for each field of view all light-sensitive sensor elements of the camera can be used or the first and second field of views are realized by reading out respective sub-groups of light-sensitive sensor elements of the array of light-sensitive sensor elements.

The array of light-sensitive sensor elements may specifically be arranged as a regular M times N matrix of RGB light-sensitive detector elements. The portion of the spacer attachment that extends into the first field of view may be a portion of the contact portion, specifically at least one portion of the upper free end of the contact portion, but it is also possible that at least one projection is provided at the oral scanner attachment / spacer attachment, preferably at the contact portion, which projection extends into the first field of view of the camera. The portion of the spacer attachment extending into the first field of view may have a predetermined optical property that may be chosen for simple identification. The optical property may be a color. The color may preferably be black or blue or red or white or yellow or green etc.

In some embodiments, the light-sensitive sensor element array is provided as a rectangular M times N array of light-sensitive sensor elements, preferably where at least one light-sensitive sensor element of the rectangular M times N array is arranged to image a portion of the oral scanner attachment, further preferably where a plurality of light-sensitive sensor elements of the rectangular M times N array are arranged to image the portion of the oral scanner attachment.

While it shall not excluded that the oral scanner captures an image using the first field of view of the camera once in a while and transmits it to the processor, the oral scanner system may be arranged so that upon operation of a user-operable input element, the oral scanner system first captures an image using the first field of view and the at least one signal from the array of light-sensitive sensor elements is then transmitted to the processor to determine whether the spacer attachment is attached or not. It is noted that only a respective fraction of the signals from the array of light-sensitive sensor elements that are intended to capture the portion of the spacer attachment and potentially some signals from other light-sensitive sensor elements, e.g., from a central area of the array of light-sensitive sensor elements, may be sufficient to determine the presence of the spacer attachment. Indeed, it may be sufficient to send only one signal from one light-sensitive sensor element to the processor to enable determination of the presence of a correctly attached oral scanner attachment.

In some embodiments, the processor is arranged to receive a plurality of signals from a first group of light-sensitive sensor elements of the light-sensitive sensor element array to determine the attachment state of the spacer attachment, and the processor is further arranged to receive a plurality of signals from a second group of light-sensitive sensor elements of the light-sensitive sensor array during the scanning procedure, where the first group of light-sensitive sensor elements comprises at least one light-sensitive sensor element that is not part of the second group of light-sensitive sensor elements.

In order to capture an image with the first field of view of the camera, the oral care system may switch on the first light source or another light source to illuminate the portion of the spacer attachment that extends into the maximum field of view of the camera and potentially to illuminate any other objects in front of the camera, e.g., parts of the oral cavity as a user may typically only want to initiate a scanning procedure if the head of the oral scanner is already positioned in the oral cavity. But it may not be necessary to switch on a light source. It can also be assumed that enough ambient light is present so that at least any objects in front of the camera are brighter than the portion of the spacer attachment extending into the maximum field of view of the camera. It also contemplated that the oral scanner system captures one image with the first or other light source switched on and one image without switching on any light source.

The processor is arranged to determine whether the spacer attachment is present by comparing a value of the signal of at least one light-sensitive sensor element that is intended to image the portion of the spacer attachment that extends into the field of view of the camera with a threshold value, preferably the processor determines whether the signal is below or above a threshold value and thus whether the light intensity is particularly low or high and thus indicates the presence of the portion of the spacer attachment. The processor may do the same comparison for a plurality of signals that relate to light-sensitive sensor elements intended to image the said portion of the spacer attachment or the processor may combine and/or average the signals of said light-sensitive sensor elements. The processor may further be arranged to compare the at least one signal from a light-sensitive sensor element intended to image said portion of the spacer attachment with one or more, preferably averaged signals from more centrally arranged light-sensitive sensors that are not intended to image said portion of the spacer attachment and to make sure that this signal or these signals relate to a higher light intensity, preferably to a light intensity above a certain level derived from tests of the oral scanner system under various usual use conditions. The latter comparison shall make sure that the image was not taken in plain darkness, which may cause an erroneous positive determination of the presence of the spacer attachment. The above description assumed that the portion of the spacer attachment that is imaged by the camera is essentially black. The processor may then be arranged to first convert the signals from the camera, if those comprise several color channels as is the case for an RGB camera, into a grayscale image. If said portion has a different color, for example red, then the processor may be arranged to specifically analyze the R color channel of the light-sensitive sensor element(s) that is (are) intended to image said portion of the distance element. The oral scanner system may then be arranged to switch on a white light source or a light source that emits red light when the image is captured.

In case that the processor determines that the oral scanner attachment is not attached, the processor may then be arranged to indicate this attachment state to a user by a signal provided by an indicator as was already described. The processor may also be arranged to then inhibit the scanning procedure.

In some embodiments, the at least one portion of the spacer attachment that extends into the field of view of the camera comprises a visually recognizable identification feature such as a bar code, a QR code, a color or color combination and/or a symbol or symbols and the processor is arranged to analyze the at least one signal from the camera to determine the identification feature. Above it was described that the correctly attached oral scanner attachment may be identified by its specific color, e.g., red. Here, it is meant that different oral scanner attachments can be differentiated by each type having a different color, e.g., red for one type of oral scanner attachment and green for another type of oral scanner attachment. Of course, the color is irrelevant if there is a different visually recognizable identification feature such as a bar code.

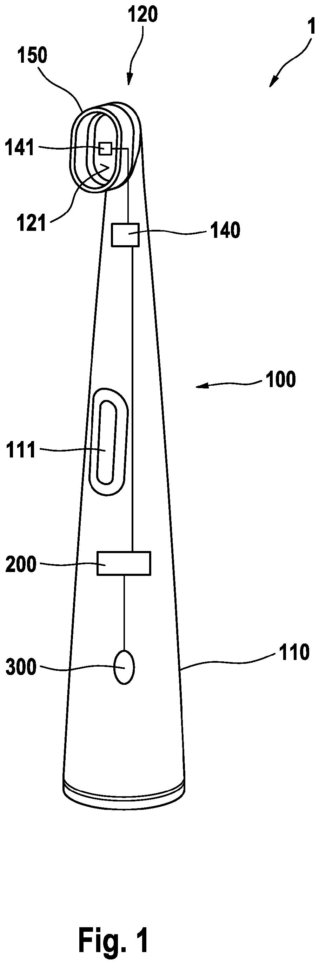

Fig. 1 is a depiction of an example oral scanner system 1 comprising an oral scanner 100 and a processor 200 that is provided within a handle 110 of the oral scanner 100, i.e., the oral scanner system 1 is realized as a single handheld device. The oral scanner 100 comprises the handle 110 and a head 120. A user-operable input element 111 such as an on/off button may be realized at the handle 110. While the handle 110 has a size so that it can be easily grasped by a hand of a human user, the head 120 is smaller in size and dimensioned so that it can be easily introduced into the oral cavity of a human to perform a scanning procedure of at least a portion of the oral cavity, preferably a scanning procedure covering the complete dentition or some teeth or a single tooth or the tongue etc. A light inlet 141 of a camera 140 is provided at an essentially planar front surface 121 of the head 120. The oral scanner 100 may comprise a transceiver 300 for at least receiving data such as new firmware software or the like and for potentially also transmitting data such as processed data that can be send to an external display device where the user may then be able to see live images from the camera 140 and/or processed data such as the plaque level per location in the oral cavity.

A spacer attachment 150 is attached to the head 120. As was already explained, the spacer attachment 150 serves to allow a user to maintain an essentially constant distance between the camera 140 and/or the light inlet 141 and the objects to be scanned and further the spacer attachment 150 is here shaped to effectively block ambient light from reaching the camera 140 via the light inlet 141. The distance that is defined by the spacer attachment 150 when being in contact with a surface of an object to be scanned may coincide with a focal length of the camera so that sharp images can be captured when the oral scanner operates while the spacer attachment 150 is in contact with the object(s) to be scanned. While not shown, the head 120 may also comprise a light source that may comprise a light outlet in the essentially planar front surface 121 so that the objects to be scanned may then be essentially solely illuminated by the light of the light source.

The oral scanner 100 may, inter alia, comprise a display (not shown) or any other indicator for visually, haptically and/or audibly conveying a signal perceivable by a user.

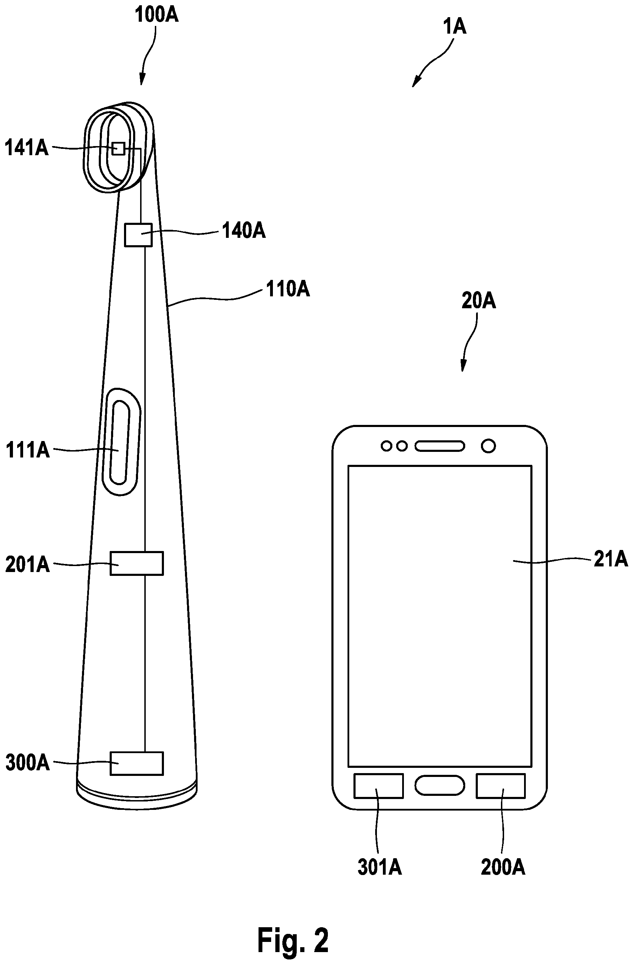

Fig. 2 is a depiction of an oral scanner system 1A comprising an oral scanner 100A and a processor 200A that is provided by a separate device 20A, here shown to be a smartphone. It is referred to the description of Fig. 1 for the other features of the oral scanner 100A that are identical to the oral scanner 100 of Fig. 1. The processor 200A may be connected with a transceiver 300A to wirelessly transmit data to and receive data from a remote computing device, e.g., via a router in a home network. The processor 200A may then be understood as a first processor that is arranged to outsource certain processing steps to a second processor (not shown) provided by the remote computing device representing a second processor. Alternatively, a processor 201A may be provided by the oral scanner 100A that may be understood as a second processor. It is noted that in this context "first" and "second" bear no particular meaning other than to differentiate between physically separate processor portions that together realize the processor of the oral care system. There may also be more processors, e.g., a third processor in a remote computing device. The separate device 20A may, inter alia, comprise a display 21A or any other indicator for visually, haptically and/or audibly conveying a signal perceivable by a user.

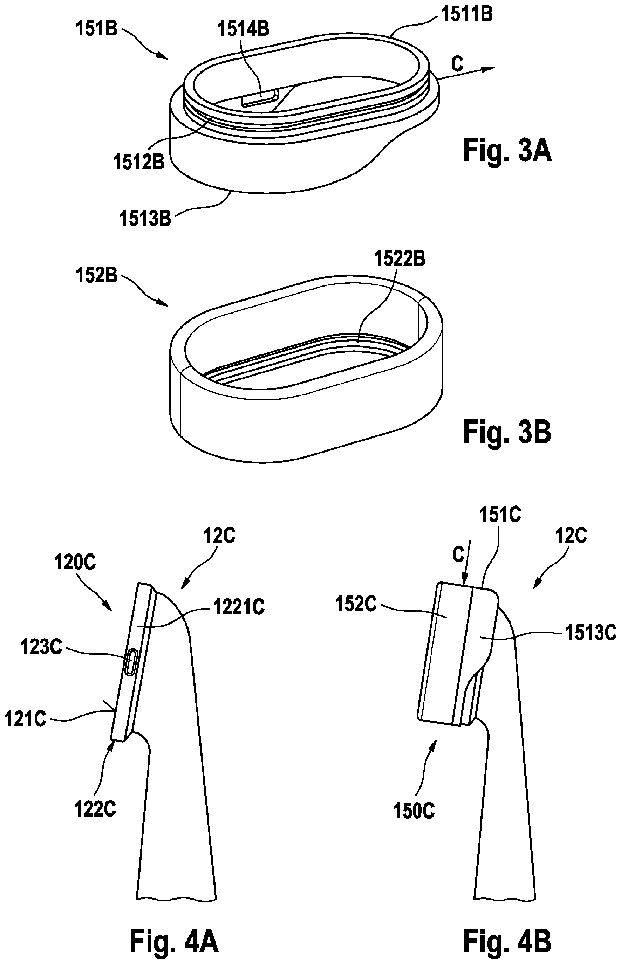

Figs. 3A and 3B show depictions of an example connector portion 151B of a spacer attachment and of a contact portion 152B of the spacer attachment, respectively. While the connector portion 151B and the contact portion 152B are here shown isolated from each other for sake of visibility of details, it is to be understood that the connector portion 151B and the contact portion 152B may be non-detachably connected with each other, e.g., the contact portion 152B may be made from an elastomeric material that is injection molded onto the connector portion 151B, which in turn may be made by injection molding from a thermoplastic material. In the shown embodiment, the connector portion 151B comprises a connector wall 1511B via which the contact portion 152B is connected with the connector portion 151B. If the contact portion 152B is injection molded onto the connector portion 151B, the materials may be chosen such that they chemically bond to each other. Further, as is shown, the connector portion 151B may comprise one or several undercut structures such as a ring-like depression 1512B for providing a positive mechanical engagement between the two portions.

In Fig. 3A it can be seen that the connector portion comprises a U-shaped connector 1513B that can be slid onto ahead of an oral scanner along direction C. The U-shaped connector 1513B may comprise one or more mechanical engagement elements 1514B. In the shown embodiment, a mechanical engagement element 1514B is realized as a depression that may engage with a similarly shaped projection provided at the head of the oral scanner (see Fig. 4A). The connector portion 151B may comprise a second mechanical engagement element opposite to the mechanical engagement element 1514B on the other side of the U-shape connector 1513B.

In Fig. 3B the contact portion 152B is realized as a lozenge-shaped closed wall element. A ring-like projection 1522B is in a connected state mechanically engaged with the ring-like depression 1512B.

Figs. 4A and 4B are depictions of a head portion 12C of an oral scanner in a side view, where in Fig. 4A the head portion 12C is shown without a spacer attachment and in Fig. 4B a spacer attachment 150C is attached to the head 120C of the oral scanner. Without wanting to be limiting, the spacer attachment 150C may comprise a connector portion 151C and a contact portion 152C as shown in Figs. 3A and 3B. The head 120C of the oral scanner here comprises a plate-like portion 122C that may provide an essentially flat front surface 121C as was discussed in connection with Fig. 1. The plate-like portion 122C may be lozenge-shaped when seen in top view. Further, the plate-like portion 122C here comprises a mechanical engagement element 123C in the form of a projection located on a side-edge 1221C of the plate-like portion 122C.

The mechanical engagement element 123C is arranged and provided to cooperate with a mechanical engagement element of the spacer attachment to enable a secure fixation of the spacer attachment at the head 120C of the oral scanner. Fig. 4B shows the head portion 12C with the spacer attachment 150C being attached to the head 120C. The spacer attachment 150C here comprises a U-shaped connector 1513C that was slid onto the plate-like portion 122C of the head 120C along a connecting direction C until the mechanical engagement elements of the spacer attachment 150C and of the head 120C came into engagement. The contact portion 152C of the spacer attachment 150C may here be realized as a lozenge-shaped closed wall element surrounding the head 120C, specifically surrounding a light inlet for a camera provided at the head 120C and optionally at least one light outlet.

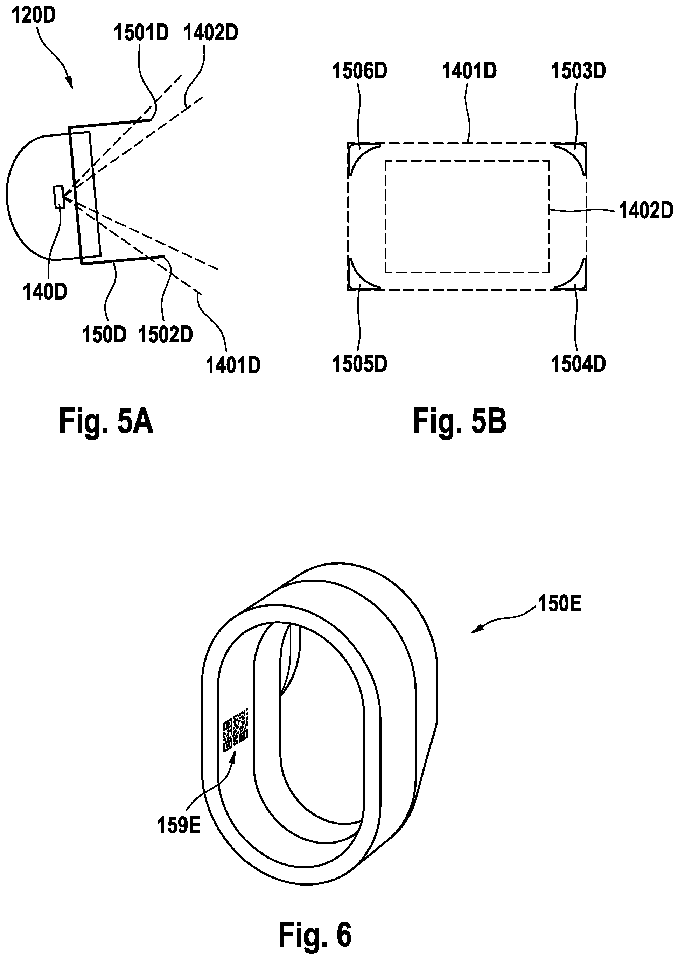

It may be sensible for a secure use and for enabling a quality scanning procedure to ensure that the spacer attachment is attached to the head of the oral scanner and in particular that the spacer attachment is correctly attached to the head. In accordance with the present disclosure, an attachment state of the spacer attachment is determined by analyzing data acquired by the camera, i.e., the attachment state of the spacer attachment is visually determined. The camera may for this visual detection have a first field of view for the determination of the attachment state of the spacer attachment and a second field of view for the scanning procedure that are different. Specifically, the camera of the oral scanner may comprise an array of light-sensitive sensor elements where at least one of the light-sensitive sensor elements is part of the first field of view. A processor of the oral scanner system may be structured and arranged to specifically read out or receive signals only from the at least one light-sensitive sensor element that is assigned to the first field of view for the determination of the attachment state of the spacer attachment. The first field of view may comprise only light-sensitive sensor elements that are not part of the light-sensitive sensor elements of the second field of view or the light-sensitive sensor elements may at least partly overlap for the first and the second field of view or the first field of view may comprise all light-sensitive sensor elements of the second field of view. Some details of the visual detection of the attachment state of the spacer attachment are discussed in the following with reference to Figs. 5A and 5B.

Fig. 5A is a simplified and schematic cross-sectional cut through a head 120D of an oral scanner comprising a spacer attachment 150D where a first field of view 1401D and a second field of view 1402D of a camera 140D are indicated. The second field of view 1402D may be the field of view used for performing a scanning procedure with the oral scanner. The first field of view 1401D is here shown to be wider than the second field of view 1402D and may be a maximum field of view of the camera 140D even though it is not necessary that the first field of view 1401D is indeed representing the maximum field of view of the camera 140D. It is in this example relevant that at least one portion 1501D, 1502D of the spacer attachment 150D projects into the first field of view 1401D.

Fig. 5B is a schematic depiction of a first field of view 1401D and of a second field of view 1402D, where the second field of view 1402D lies within the first field of view 1401D. But this shall not exclude that the first field of view is realized by the rectangular ring outside of the second field of view 1402D. In case the optics of the camera are not changed, the field of view is flexible as it is essentially given by the light-sensitive sensor elements of the array of light-sensitive sensor elements that are being read out - see also next paragraph. It is indicated that portions of a spacer attachment 1503D, 1504D, 1505D and 1506D project into the first field of view 1401D when the spacer attachment is in its attached state. In case that the spacer attachment is missing, no such portions of the spacer attachment would project into the first field of view 1401D. In case that the spacer attachment would have been wrongly attached, e.g., would not have been completely slid onto the head of the oral scanner, the portions of the spacer attachment would asymmetrically project into the first field of view 1401D in contrast to the symmetric situation as shown in Fig. 5B.

In the context of the discussion of the field of view of the camera of the oral scanner, it is noted that a field of view of the camera is defined by the array of light-sensitive sensor elements and the optical elements such as lenses of the camera. It is thus obvious that a discussion involving two different fields of view of a single camera with fixed optical elements means a selection of light-sensitive sensor elements that are read out. But this shall not exclude that the camera of the oral scanner has adaptable optics like a zoom in / zoom out optics that change the field of view of the camera by modifications of the optics of the camera. The terms "field of view", "first field of view" and "second field of view" are thus independent from the technology used to provide the fields of view. In addition, "field of view" shall also refer to a portion of the real field of view seen by the camera, e.g., the first field of view may be defined to be a sub-group of light-sensitive sensor elements that are selected as at least one of these light-sensitive sensor elements will image at least a portion of the spacer attachment when it is in the attached state.

Fig. 6 is a depiction of a spacer attachment 150E that comprises a visually detectable identifier 159E that here as an example is shown to be a QR code. The identifier 159E is located on the spacer attachment 150E such that it can be imaged by the camera of the oral scanner when the spacer attachment 150E is in its attached state. The processor may then be arranged to determine the type of identifier 159E and specifically to set a scanning parameter and/or to adapt a control of the scanning procedure in dependence on the type of identifier 159E. The identifier 159E may convey the type of the spacer attachment, e.g., the focal length or the softness of the closed wall element or the like. The identifier 159E may additionally or alternatively allow a personalization by assigning a certain type of spacer attachment to a specific user. E.g., different spacer attachments may be provided in different colors, which color is then a visually differentiable identifier and a user may assign a respective color to his or her name so that, e.g., personalized settings can be used, or the scan results may then be assigned to a personal account etc. Instead of a QR code or bar code or a color, other visually detectable features may be used as identifier 159E, e.g., a projection extending from a corner of the spacer attachment or a plurality of such projections extending from different corners may allow to differentiate between 2 to the power of 4, i.e., 16 different spacer attachments.

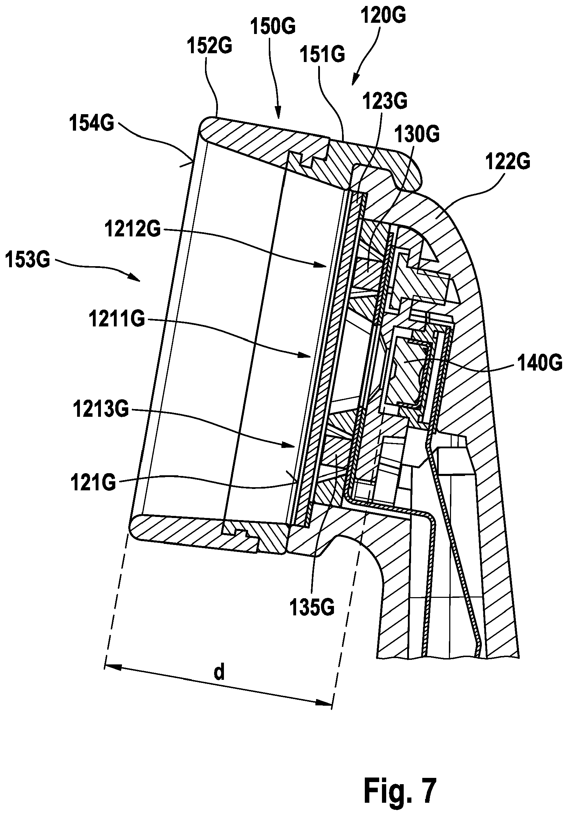

Fig. 7 is a cross-sectional cut through a head 120G of an oral scanner. The head 120G comprises an essentially flat front surface 121G and a head housing 122G as well as a first light source 130G, a second light source 135G and a camera 140G. The essentially flat front surface 121G here comprises a window 123G that may be realized as a sheet of glass or plastic that is essentially transparent for the light emitted by the first and second light sources 130G and 135G and that is also essentially transparent for the fluorescence light that shall be captured by the camera (specifically the green and red fluorescence light mentioned before). "Essentially transparent" shall mean that at least 80% of the relevant light passes through the window, specifically passes through the window without being scattered. In the shown embodiment, the head 120G comprises a light inlet 1211G aligned with the camera 140G, a first light outlet 1212G aligned with the first light source 130G and a second light outlet 1213G aligned with the second light source 135G. A spacer attachment 150G is attached to the housing 122G of the head 120G. The spacer attachment 150G comprises a connector portion 151G and a contact portion 152G. The contact portion 152G is realized as a closed wall element that defines an aperture 153G and a closed contact surface 154G at the free top end of the contact portion 153G. The spacer attachment 150G defines a distance d between the contact surface 154G and a light sensitive sensor array of the camera 140G that coincides with a focal length of the camera 140G.

While the above-described embodiments focus on the detection of an attachment state of a spacer attachment at a head of an oral scanner by means of an optical detection that uses a camera of the oral scanner, which camera is specifically used for performing an optical scanning procedure of at least a portion of the oral cavity, the present invention is not limited to an oral scanner comprising a camera. As a matter of fact, the oral scanner may use any other scanning technique(s) and any other oral scanner attachment may be used with such an oral scanner. Thus, the attachment state of an oral scanner attachment can also be determined in a different manner. Basically, it is envisaged that the head of the oral scanner comprises a first interacting element, which might in some embodiments be a camera, and that the oral scanner attachment comprises a second interacting element, which might in some embodiments be at least one portion of the oral scanner attachment that is located within a field of view of the camera when the oral scanner attachment is properly, i.e., correctly attached. Some oral scanners may be used to perform a capacitive scanning of at least a portion of the oral cavity or may comprise any other sensor for performing a scanning procedure such as a pH sensor, a malodor sensor etc. Instead of a camera and a portion of the oral scanner attachment being imaged, the first and second interacting elements may be differently realized. E.g., the first interacting element may be an RFID reader and the second interacting element may be an RFID chip. The first interacting element may be a Hall sensor and the second interacting element may be a permanent magnet. The first interacting element may be at least one switch and the second interacting element may be at least one projection that activates the switch in the attached state. The first interacting element may comprise at least one light source and at least one light detector and the second interacting element may comprise at least one mirror or at least one element that enters into the light path between the light source and the light sensor in the attached state. The oral scanner attachment may be a spacer attachment, a protective attachment or any other attachment that can be used with an oral scanner. The above discussion shall not exclude that a camera is present at the oral scanner but that the attachment state of the oral scanner attachment is determined by a different technology than imaging by the camera.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

1. An oral scanner system comprising
an oral scanner having
a handle;
a head;
a camera having a light inlet provided at the head, the camera comprising a light-sensitive sensor element array arranged for outputting at least one signal indicative of a light intensity on at least one of a plurality of light-sensitive sensor elements of the light-sensitive sensor element array;
a spacer attachment being detachably attached to the head, the spacer attachment enabling a constant distance between a scanned object and the light inlet of the camera; and a processor being arranged to receive the at least one signal from the camera to determine, based on an analysis of the at least one signal, an attachment state of the spacer attachment.

2. The oral scanner system in accordance with claim 1, wherein the processor is provided in a separate device, and the oral scanner comprises a transmitter or a transceiver for transmitting the at least one signal or a processed signal derived from the at least one signal and the separate device comprises a receiver or a transceiver for receiving the at least one signal or the processed signal.

3. The oral scanner system in accordance with claim 1 or claim 2, wherein the processor is arranged to initiate an action in dependance on the determined attachment state of the spacer attachment such as to inhibit the scanning procedure if the analysis of the at least one signal reveals that the spacer attachment is not attached.

4. The oral scanner system in accordance with claim 3, wherein the action is an indication of the determined attachment state of the spacer attachment via an indicator such as a visual indicator, an acoustic indicator and/or a tactile indicator.

5. The oral scanner system in accordance with one of claims 1 to 4, wherein the spacer attachment has a contact portion intended for contacting a surface within an oral cavity, preferably wherein the contact portion is realized as a closed wall defining an aperture, further preferably wherein the closed wall is ring-shaped, O-shaped, lozenge-shaped, ellipsoidal, oval or essentially rectangular, and even further preferably wherein the closed wall surrounds the light inlet of the camera.

6. The oral scanner system in accordance with one of claims 1 to 5, wherein the spacer attachment has a U-shaped connector portion for detachable connection with a connector portion of the head of the oral scanner, preferably wherein a connection direction for connecting the U-shaped connector portion is essentially coinciding with a longitudinal extension direction of the oral scanner.

7. The oral scanner system in accordance with one of claims 1 to 6, comprising a user-operable input element for initiating a scanning procedure by the oral scanner and the processor being arranged to receive the at least one signal from the camera in response to an operation of the user-operable input element.

8. The oral scanner system in accordance with one of claims 1 to 7, wherein the spacer attachment comprises at least one portion that extends into a field of view of the camera when the spacer attachment is attached to the head of the oral scanner.

9. The oral scanner system in accordance with claim 8, wherein the light-sensitive sensor element array is provided as a rectangular M times N array of light-sensitive sensor elements, preferably where at least one light-sensitive sensor element of the rectangular M times N array is arranged to image the portion of the spacer attachment, further preferably where a plurality of light-sensitive sensor elements of the rectangular M times N array are arranged to image the portion of the spacer attachment.

10. The oral scanner system in accordance with one of claims 8 to 9, wherein the processor is arranged to use a sub-plurality of light-sensitive sensor elements from the rectangular M times N array of light-sensitive sensor elements for performing the scanning procedure, which sub-plurality of light-sensitive sensor elements does not include the at least one light-sensitive sensor element that images the portion of the spacer element.

11. The oral scanner system in accordance with one of claims 8 to 10, wherein the at least one portion of the spacer attachment that extends into the field of view of the camera has a predetermined visually detectable property such as a predetermined color.

12. The oral scanner system in accordance with one of claims 1 to 11, wherein the processor is arranged to receive a plurality of signals from a first group of light-sensitive sensor elements of the light-sensitive sensor element array to determine the presence of the spacer attachment, and the processor is further arranged to receive a plurality of signals from a second group of light-sensitive sensor elements of the light-sensitive sensor array during the scanning procedure, where the first group of light-sensitive sensor elements comprises at least one light-sensitive sensor element that is not part of the second group of light-sensitive sensor elements.

13. The oral scanner system in accordance with one of claims 1 to 12, wherein the processor is arranged to determine whether the spacer attachment is attached by comparing a value of the at least one signal with at least one threshold value or by determining whether the value of the at least one signal of the at least one light-sensitive sensor element lies within a predetermined signal value range or by determining whether the value of the at least one signal differs from a value of at least one further signal received from the camera.

14. The oral scanner system in accordance with one of claims 1 to 13, wherein the spacer attachment comprises a visually recognizable identification feature such as a bar code, a QR code, a color or color combination and/or a symbol or symbols and the processor is arranged to analyze the at least one signal from the camera to determine the identification feature.

15. An oral scanner system comprising
an oral scanner having
a handle;
a head;
an oral scanner attachment being detachably attached to the head;
wherein the head comprises a first interacting element and the oral scanner attachment comprises a second interacting element, the first interacting element and the second interacting element being arranged so that the first interacting element provides at least one signal being indicative of an attachment state of the oral scanner attachment in dependence on the presence of the second interacting element;
and
a processor being arranged to receive the at least one signal from the first interacting element and to determine the attachment state of the oral scanner attachment and preferably further being arranged to initiate an action in dependence on the determined attachment state such as an indication of the determined attachment state via an indicator such as a visual indicator, an acoustic indicator and/or a tactile indicator.
